# FASCICULE DE BREVET EUROPEEN

(11) **EP 2 268 601 B1**
(45) Date de publication et mention de la délivrance du brevet: **17.09.2014**
(21) Numéro de dépôt: 09723286.2
(22) Date de dépôt: 09.03.2009
(51) Int. Cl.: C07C 51/04, C07C 53/18, C07C 51/58, C07C 53/48

(54) **PROCEDE DE PREPARATION DE L'ACIDE DIFLUOROACETIQUE ET DE SES SELS**
VERFAHREN ZUR HERSTELLUNG VON DIFLUORESSIGSÄURE UND SALZEN DARAUS
METHOD FOR PREPARING DIFLUOROACETIC ACID AND SALTS THEREOF

(30) Priorité: 19.03.2008 FR 0801513
(43) Date de publication de la demande: 05.01.2011
(73) Titulaire: Rhodia Opérations, 75009 Paris (FR)
(72) Inventeur: METZ, François, 69540 Irigny (FR); SAINT-JALMES, Laurent, 69390 Vourles (FR)
(74) Mandataire: Dutruc-Rosset, Marie-Claude
(86) Numéro de dépôt international: PCT/EP2009/052740
(87) Numéro de publication internationale: WO 2009/115426

(56) Documents cités:
- EP-A- 0 694 523
- EP-A- 0 928 783
- WO-A-96/29298
- GB-A- 976 316
- US-A1- 2005 148 649

## Description

La présente invention a pour objet un procédé de préparation de l'acide difluoroacétique et de ses sels.

L'invention vise également la préparation du fluorure de difluoroacétyle qui est utilisé comme produit intermédiaire de fabrication de l'acide difluoroacétique.

L'invention se rapporte plus précisément à un procédé de préparation du fluorure de difluoroacétyle, en phase gazeuse.

Il existe différents modes de préparation de l'acide difluoroacétique ou de ses sels.

En particulier, il est connu de préparer l'acide difluoroacétique selon une réaction d'échange chlore-fluor, en faisant réagir le fluorure de potassium sur des dérivés de l'acide dichloroacétique, par exemple, le N,N-diéthylchlorofluoroacétamide (Chemical Abstracts 88, (1), 6300m) ou le N-pyrrolidine-chlorofluoroacétamide (Chemical Abstracts 108, (19), 166949q).

Un mode d'obtention tout à fait différent de l'acide difluoroacétique, réside dans une réaction d'oxydation du difluoroéthanol obtenu par hydrolyse du 1-bromo-2,2-difluoroéthane [Y. Désirant, Bull. sci. acad. roy. Belg. [5] 15, 966-82 (1929)].

Il est encore connu de la demande de brevet WO 96/29298 de préparer de l'acide difluoroacétique par oxydation de 1,1-dichloro-2,3-difluoroéthane.

Une autre voie de synthèse est fondée sur une réaction de déshydrohalogénation à partir de l'acide monochlorodifluoroacétique. On peut citer en particulier US 5 455 376 qui décrit l'obtention de l'acide difluoroacétique par hydrogénation en phase gazeuse de l'acide monochlorodifluoroacétique, en présence d'un catalyseur à base d'un métal noble, palladium ou platine déposé sur support, par exemple l'alumine.

Il existe également un procédé de préparation décrit dans EP 1137615, par hydrogénation en phase liquide et milieu sodique de l'acide monochlorodifluoroacétique, en présence de nickel de Raney. Le rendement chimique est bon mais la productivité d'un tel procédé est à améliorer. Il peut y avoir formation comme sous-produit, de l'acide monofluoroacétique qui est un produit particulièrement toxique. Par ailleurs, l'acide obtenu est sous une forme sodique, difficile à récupérer à partir du milieu réactionnel.

L'invention a pour but de proposer un procédé de préparation de l'acide difluoroacétique faisant appel à un substrat de départ de nature différente.

Il a maintenant été trouvé et c'est ce qui constitue l'objet de la présente invention un procédé de préparation de l'acide difluoroacétique caractérisé par le fait qu'il comprend une étape de préparation du fluorure de difluoroacétyle par réaction du chlorure de dichloroacétyle et de l'acide fluorhydrique, en phase gazeuse, en présence d'un catalyseur à base de chrome suivie par une étape d'hydrolyse du fluorure de difluoroacétyle obtenu.

Un autre objet de l'invention est le procédé de préparation du fluorure de difluoroacétyle.

Un autre objet de l'invention est le procédé de préparation de sels de l'acide difluoroacétique.

Intervient dans le procédé de l'invention, un catalyseur de fluoration à base de chrome.

Le catalyseur utilisé comprend de préférence les oxydes, halogénures, oxyhalogénures ou sels minéraux de chrome éventuellement dopé par un élément métallique tel que par exemple le nickel, le cobalt, le magnésium et le zinc.

Il s'agit préférentiellement d'un oxyde de chrome, d'un fluorure de chrome ou d'un oxyfluorure de chrome ou bien du chrome dopé par un élément métallique comme par exemple, le nickel et le magnésium.

Le catalyseur peut subir une activation par un traitement thermique et/ou un traitement de fluoration. En particulier, l'activation peut avoir lieu lors de la fluoration. La température est avantageusement choisie entre 100°C et 400°C, de préférence entre 200°C et 300°C.

On utilise en particulier le chrome sous forme d'oxydes sous différents degrés d'oxydation et/ou sous forme d'hydroxydes sous forme de poudre ou de gel.

On peut mettre en oeuvre un oxyde de chrome III activé préparé par exemple par précipitation de sels de chrome III hydrosolubles comme par exemple, les chlorures, nitrates, acétates, sulfates à l'aide d'un hydroxyde de métal alcalin, de préférence le sodium ou le potassium ou d'ammonium. Le précipité est séché à environ 110°C et calciné à une température inférieure à 700°C, de préférence comprise entre 400 et 600°C.

L'oxyde de chrome anhydre peut être obtenu par calcination de sels de chrome inorganiques comme le chromate d'ammonium ou le nitrate de chrome ou par calcination de sels de chrome organiques comme par exemple, les oxalates ou formiates de chrome à 350°C, sous atmosphère d'azote.

On peut faire appel notamment à un catalyseur de type Cr-Ni, avec une valence du chrome comprise entre 2 et 3 et celle du nickel comprise entre 0 et 2, la quantité de nickel exprimée en % atomique représentant de 0,1 à 10 %.

Un mode de préparation de ce catalyseur consiste à faire une décomposition thermique, séparément ou en mélange d'un ou plusieurs sels organiques du chrome (par exemple oxalate) et d'un sel ou plusieurs sels du nickel (par exemple oxalate), mise en forme du mélange puis fluoration du catalyseur mis en forme.

La décomposition thermique a lieu généralement entre 370°C et 400°C, sous atmosphère de gaz inerte, par exemple l'azote.

La mise en forme du catalyseur obtenu peut être faite, dans des conditions non oxydantes, par exemple par extrusion puis le produit mis en forme est séché vers 120°C - 130°C, puis calciné à 370°C - 400°C, sous atmosphère inerte.

Le catalyseur est chauffé entre 100°C et 500°C, sous acide fluorhydrique entre 1 et 12 heures.

Un catalyseur de type Cr-Mg peut également être mis en oeuvre.

Il peut être obtenu notamment par mélange d'un sel de chrome (par exemple nitrate) en solution avec un oxyde ou hydroxyde de magnésium, séchage prolongée entre 12 et 24 heures par exemple à 100°C, puis activation par l'acide fluorhydrique, par exemple à 200°C.

La phase active peut être apportée sous une forme finement divisée ou bien mise en forme ou déposé sur un support.

Comme exemples de supports, on peut mentionner la silice, l'alumine, la zircone, l'oxyde de titane. De préférence, le chrome est déposé sur un support à raison de 0,5 % à 5 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Conformément au procédé de l'invention, on effectue dans une première étape, une réaction de fluoration en faisant réagir le chlorure de dichlorocétyle et l'acide fluorhydrique, en phase gazeuse, en présence du catalyseur de fluoration.

Le rapport entre l'acide fluorhydrique et le chlorure de dichloroacétyle peut varier largement. Généralement, la quantité de l'acide fluorhydrique est excédentaire. Ainsi, le rapport entre le nombre de moles d'acide fluorhydrique et le nombre de moles de chlorure de dichloroacétyle varie le plus souvent entre 1 et 30. Il est avantageusement choisi entre 6 et 10.

On réalise le procédé conforme à l'invention à une température élevée, en règle générale supérieure à 200°C. Il est recommandé de travailler à des températures comprises entre 250°C et 400°C, de préférence comprise entre 250°C et 300°C.

Pour des raisons de simplicité, on réalise le procédé de l'invention sous la pression atmosphérique. Toutefois, il est également possible d'opérer sous des pressions plus basses ou plus élevées.

D'un point de vue pratique, le procédé peut être mis en oeuvre en discontinu ou en continu.

On commence par effectuer le mélange d'une façon quelconque, du chlorure de dichloroacétyle et de l'acide fluorhydrique.

Ainsi, on peut mélanger lesdits réactifs, dans une zone de mélange, puis envoyer le mélange obtenu sur le lit catalytique.

Lorsque l'on réalise le procédé en discontinu, la quantité de catalyseur de fluoration mis en oeuvre, exprimée en poids de catalyseur par poids du chlorure de dichloroacétyle peut varier, par exemple, entre 0,5 et 20 %, de préférence entre 0,5 et 5 %.

L'autre variante de l'invention consiste à conduire la réaction en continu, dans un réacteur tubulaire comportant le catalyseur solide disposé en lit fixe.

Le chlorure de dichloroacétyle et l'acide fluorhydrique peuvent être introduits séparément ou en mélange dans le réacteur. Comme mentionné précédemment, on peut les mélanger dans une zone de mélange, puis envoyer le mélange obtenu sur le lit catalytique.

Le mélange réactionnel traverse le lit catalytique, de préférence, de bas en haut.

Le temps de contact qui est défini comme le rapport entre le volume apparent de catalyseur et le débit du flux gazeux peut varier largement, et est le plus souvent compris entre 0,2 et 100 secondes. Le temps de contact est choisi de préférence entre 5 et 50 secondes.

Le poids de substrat mis en oeuvre par poids de catalyseur et par heure varie généralement entre 0,01 h⁻¹ et 2 h⁻¹, de préférence entre 0,05 h⁻¹ et 0,5 h⁻¹.

En fin de réaction, on récupère une phase gazeuse comprenant le fluorure de difluoroacétyle, l'acide fluorhydrique en excès, l'acide chlorhydrique formé par la réaction.

Conformément au procédé de l'invention, on effectue ensuite une étape d'hydrolyse du fluorure de difluoroacétyle en acide difluoroacétique.

A cet effet, on met le flux gazeux en présence d'eau. La quantité d'eau mise en oeuvre est au moins égale à la quantité stoechiométrique.

Généralement, l'opération est effectuée en envoyant le flux gazeux dans une colonne d'hydrolyse : l'eau étant envoyée à contre-courant du flux gazeux qui remonte de bas en haut dans la colonne.

On peut également effectuer une hydrolyse acide, par exemple en faisant appel à une solution d'un acide minéral fort, par exemple de l'acide chlorhydrique à 30 % en poids.

Ainsi, on récupère en pied de colonne, l'acide difluoroacétique et en tête de colonne, l'acide chlorhydrique gazeux.

Les sels de l'acide difluoroacétique peuvent être aisément fabriqués à partir de l'acide, notamment par réaction avec une base, de préférence la soude ou la potasse.

Le procédé de l'invention est avantageusement conduit dans un appareillage susceptible de résister à la corrosion provoquée par l'acide fluorhydrique. A cet effet, on choisit des matériaux comme par exemple des aciers vitrifiés ou des aciers Hastelloy^{®}.

Selon une variante de l'invention, on récupère le fluorure de difluoroacétyle à partir dudit flux gazeux comprenant le fluorure de difluoroacétyle, l'acide fluorhydrique en excès, l'acide chlorhydrique formé par la réaction, en condensant ledit flux gazeux par abaissement de sa température entre - 40°C à 10°C, de préférence entre -20°C à 0°C puis en effectuant la distillation du flux condensé.

Le procédé de l'invention est particulièrement intéressant car il met en oeuvre une matière première bon marché et possède une bonne productivité.

Les exemples qui suivent, illustrent l'invention sans toutefois la limiter.

Les abréviations ont les significations suivantes :
- CDCA : chlorure de dichloroacétyle
- FDFA : fluorure de difluoroacétyle

Dans les exemples, on définit le taux de conversion et les rendements obtenus.

Le taux de conversion (TT) correspond au rapport entre le nombre de moles de substrat CDCA transformées et le nombre de moles de substrat CDCA engagées.

Le rendement (RR) correspond au rapport entre le nombre de moles de produit FDFA formées et le nombre de moles de substrat CDCA engagées.

Le rendement (RT) correspond au rapport entre le nombre de moles de produit FDFA formées et le nombre de moles de substrat CDCA transformées.

### EXEMPLES

On donne ci-après, le protocole opératoire qui est suivi dans les différents exemples.

La réaction est conduite dans un réacteur en Hastelloy C276 qui est un alliage à base de nickel (57 % en poids) comprenant 16 % de chrome, 16 % de molybdène, 5 % de fer, 4 % de tungstène, 2,5 % de cobalt, 1 % de manganèse, 0,35 % de vanadium, 0,08 % de silicium, 0,01 % de carbone.

Dans un réacteur en Hastelloy C276 constitué d'un tube de 60 cm de longueur et de diamètre extérieur de 2,5 cm, rempli d'un catalyseur à base d'oxyde de chrome III (∼150 g) préalablement séché jusqu'à poids constant et fluoré (24 heures à 250°C), on introduit du chlorure de dichloroacétyle (CDCA) préalablement distillé à un débit de 0,11 mol/h (∼15 g/h) et de l'HF anhydre à un débit de 1,1 à 2,6 mol/h (22 à 52 g/h).

La température est selon l'essai fixée de 200°C à 350°C, isotherme ou gradient.

Dans ces conditions, le temps de séjour tₛ varie entre 10 et 20 s.

Après réaction, le fluorure de difluoroacétyle (FDFA) est dosé en le transformant sous forme du sel de potassium de l'acide difluoroacétique.

Le flux sortant est hydrolysé dans des barboteurs à potasse montée en série, et les différents acides sont dosés sous forme de sels de potassium par chromatographie ionique.

### Exemples 1 à 9

Les exemples suivants illustrent l'effet de la température, du rapport HF/CDCA et du temps de séjour.

**Tableau (I)**

| Réf. ex | T°C | tₛ | HF/ CDCA | TT_{CDCA} % | RR_{FDFA} % | RT_{FDFA} % |
|---|---|---|---|---|---|---|
| 1 | 250 | 20 | 11,3 | 66 | 30 | 45 |
| 2 | 250 | 20 | 11,7 | 68 | 33 | 48 |
| 3 | 250 | 20 | 11,6 | 72 | 32 | 45 |
| 4 | 200 | 20 | 10,2 | 22 | 3 | 14 |
| 5 | 200 | 20 | 11,4 | 18 | 3 | 17 |
| 6 | 200 à 250 | 20 | 11,5 | 32 | 17 | 53 |
| 7 | 300 | 11 | 18,5 | 40 | 5 | 12,5 |
| 8 | 300 | 20 | 10 | 60 | 20 | 33 |
| 9 | 350 | 19 | 10 | 85 | 20 | 25 |

## Revendications

1. Procédé de préparation de l'acide difluoroacétique **caractérisé par le fait qu'**il comprend une étape de préparation du fluorure de difluoroacétyle par réaction du chlorure de dichloroacétyle et de l'acide fluorhydrique, en phase gazeuse, en présence d'un catalyseur à base de chrome suivie par une étape d'hydrolyse du fluorure de difluoroacétyle obtenu.

2. Procédé selon la revendication 1 **caractérisé par le fait que** le catalyseur de fluoration comprend les oxydes, halogénures, oxyhalogénures ou sels minéraux de chrome éventuellement dopé par un élément métallique.

3. Procédé selon la revendication 2 **caractérisé par le fait que** le catalyseur de fluoration comprend un oxyde de chrome, un fluorure de chrome ou un oxyfluorure de chrome ou bien du chrome dopé par un élément métallique.

4. Procédé selon l'une des revendications 1 à 3 à **caractérisé par le fait que** le catalyseur subit une activation par un traitement thermique et/ou un traitement de fluoration : l'activation pouvant avoir lieu lors de la fluoration.

5. Procédé selon l'une des revendications 1 à 4 **caractérisé par le fait que** le catalyseur est un oxyde de chrome III ayant éventuellement subi une activation par un traitement de fluoration.

6. Procédé selon l'une des revendications 1 à 5 **caractérisé par le fait que** la phase active est apportée sous une forme finement divisée ou bien mise en forme ou déposée sur un support.

7. Procédé selon la revendication 6 **caractérisé par le fait que** le chrome est déposé sur un support à raison de 0,5 % à 5 % du poids du catalyseur.

8. Procédé selon l'une des revendications 1 à 7 **caractérisé par le fait que** le rapport entre le nombre de moles d'acide fluorhydrique et le nombre de moles de chlorure de dichloroacétyle varie entre 1 et 30.

9. Procédé selon l'une des revendications 1 à 8 **caractérisé par le fait que** la température de la réaction est comprise entre 250°C et 400°C.

10. Procédé selon l'une des revendications 1 à 9 **caractérisé par le fait que** le procédé est mis en oeuvre en discontinu ou en continu.

11. Procédé selon l'une des revendications 1 à 10 **caractérisé par le fait que** la réaction est conduite en continu, dans un réacteur tubulaire comportant le catalyseur solide disposé en lit fixe.

12. Procédé selon l'une des revendications 1 à 11 **caractérisé par le fait que** l'on effectue le mélange du chlorure de dichloroacétyle et l'acide fluorhydrique dans une zone de mélange, puis l'on envoie le mélange obtenu sur le lit catalytique.

13. Procédé selon l'une des revendications 1 à 12 **caractérisé par le fait que** le temps de contact défini comme le rapport entre le volume apparent de catalyseur et le débit du flux gazeux varie entre 0,2 et 100 secondes.

14. Procédé selon l'une des revendications 1 à 13 **caractérisé par le fait que** le poids de substrat mis en oeuvre par poids de catalyseur et par heure varie entre 0,01 h⁻¹ et 2 h⁻¹.

15. Procédé selon l'une des revendications 1 à 14 **caractérisé par le fait que** l'on récupère en fin de réaction, une phase gazeuse comprenant le fluorure de difluoroacétyle, l'acide fluorhydrique en excès, l'acide chlorhydrique formé par la réaction.

16. Procédé selon l'une des revendications 1 à 15 **caractérisé par le fait que** l'on effectue ensuite une étape d'hydrolyse du fluorure de difluoroacétyle en acide difluoroacétique par mise en présence du flux gazeux avec de l'eau.

17. Procédé selon l'une des revendications 1 à 16 **caractérisé par le fait que** l'on fait réagir l'acide difluoroacétique obtenu avec une base.

18. Procédé de préparation du fluorure de difluoroacétyle **caractérisé par le fait qu'**il est obtenu par réaction du chlorure de dichloroacétyle et de l'acide fluorhydrique, en phase gazeuse, en présence d'un catalyseur à base de chrome, selon l'une des revendications 1 à 15.

## Patentansprüche

1. Verfahren zur Herstellung von Difluoressigsäure, **dadurch gekennzeichnet, dass** es einen Schritt der Herstellung von Difluoracetylfluorid durch Umsetzung von Dichloracetylchlorid und Fluorwasserstoffsäure in der Gasphase in Gegenwart eines auf Chrom basierenden Katalysators gefolgt von einem Schritt der Hydrolyse des erhaltenen Difluoracetylfluorids umfasst.

2. Verfahren nach Anspruch 1, **dadurch gekennzeichnet, dass** der Fluorierungskatalysator Oxide, Halogenide, Oxidhalogenide oder Mineralsalze von Chrom, die gegebenenfalls mit einem Metallelement dotiert sind, umfasst.

3. Verfahren nach Anspruch 2, **dadurch gekennzeichnet, dass** der Fluorierungskatalysator ein Chromoxid, ein Chromfluorid oder ein Chromoxidfluorid oder auch Chrom, das mit einem Metallelement dotiert ist, umfasst.

4. Verfahren nach einem der Ansprüche 1 bis 3, **dadurch gekennzeichnet, dass** der Katalysator eine Aktivierung durch eine Wärmebehandlung und/oder eine Fluorierungsbehandlung durchläuft, wobei die Aktivierung während der Fluorierung erfolgen kann.

5. Verfahren nach einem der Ansprüche 1 bis 4, **dadurch gekennzeichnet, dass** es sich bei dem Katalysator um ein Chrom-III-oxid, das gegebenenfalls eine Aktivierung durch eine Fluorierungsbehandlung durchlaufen hat, handelt.

6. Verfahren nach einem der Ansprüche 1 bis 5, **dadurch gekennzeichnet, dass** die aktive Phase in feinteiliger Form eingebracht oder auf einem Träger gebildet oder abgeschieden wird.

7. Verfahren nach Anspruch 6, **dadurch gekennzeichnet, dass** das Chrom in einer Menge von 0,5 bis 5 Gew.-% des Katalysators auf einem Träger abgeschieden wird.

8. Verfahren nach einem der Ansprüche 1 bis 7, **dadurch gekennzeichnet, dass** das Verhältnis zwischen der Zahl der Mole Fluorwasserstoffsäure und der Zahl der Mole Dichloracetylchlorid im Bereich zwischen 1 und 30 liegt.

9. Verfahren nach einem der Ansprüche 1 bis 8, **dadurch gekennzeichnet, dass** die Temperatur der Umsetzung zwischen 250°C und 400°C liegt.

10. Verfahren nach einem der Ansprüche 1 bis 9, **dadurch gekennzeichnet, dass** das Verfahren diskontinuierlich oder kontinuierlich durchgeführt wird.

11. Verfahren nach einem der Ansprüche 1 bis 10, **dadurch gekennzeichnet, dass** die Umsetzung in einem Rohrreaktor, der den als Festbett angeordneten festen Katalysator umfasst, kontinuierlich durchgeführt wird.

12. Verfahren nach einem der Ansprüche 1 bis 11, **dadurch gekennzeichnet, dass** man das Dichloracetylchlorid und die Fluorwasserstoffsäure in einer Mischzone mischt und die erhaltene Mischung dann auf das Katalysatorbett leitet.

13. Verfahren nach einem der Ansprüche 1 bis 12, **dadurch gekennzeichnet, dass** die als das Verhältnis zwischen dem Katalysatorschüttvolumen und der Durchflussrate des Gasstroms definierte Kontaktzeit im Bereich zwischen 0,2 und 100 Sekunden liegt.

14. Verfahren nach einem der Ansprüche 1 bis 13, **dadurch gekennzeichnet, dass** das pro Katalysatorgewicht und pro Stunde eingesetzte Substratgewicht im Bereich zwischen 0,01 h⁻¹ und 2 h⁻¹ liegt.

15. Verfahren nach einem der Ansprüche 1 bis 14, **dadurch gekennzeichnet, dass** man am Ende der Umsetzung eine Gasphase gewinnt, die das Difluoracetylfluorid, die überschüssige Fluorwasserstoffsäure und die bei der Umsetzung anfallende Chlorwasserstoffsäure umfasst.

16. Verfahren nach einem der Ansprüche 1 bis 15, **dadurch gekennzeichnet, dass** man dann einen Schritt der Hydrolyse des Difluoracetylfluorids zu Difluoressigsäure durchführt, indem man den Gasstrom mit Wasser in Kontakt bringt.

17. Verfahren nach einem der Ansprüche 1 bis 16, **dadurch gekennzeichnet, dass** man die erhaltene Difluoressigsäure mit einer Base umsetzt.

18. Verfahren zur Herstellung von Difluoracetylfluorid, **dadurch gekennzeichnet, dass** es durch Umsetzung von Dichloracetylchlorid und Fluorwasserstoffsäure in der Gasphase in Gegenwart eines auf Chrom basierenden Katalysators nach einem der Ansprüche 1 bis 15 erhalten wird.

## Claims

1. Process for preparing difluoroacetic acid, **characterized in that** it comprises a step of preparing difluoroacetyl fluoride by reacting dichloroacetyl chloride and hydrofluoric acid, in the gas phase, in the presence of a chromium-based catalyst, followed by a step of hydrolysis of the difluoroacetyl fluoride obtained.

2. Process according to Claim 1, **characterized in that** the fluorination catalyst comprises oxides, halides, oxyhalides or mineral salts of chromium optionally doped with a metal element.

3. Process according to Claim 2, **characterized in that** the fluorination catalyst comprises a chromium oxide, a chromium fluoride or a chromium oxyfluoride, or alternatively chromium doped with a metal element.

4. Process according to one of Claims 1 to 3, **characterized in that** the catalyst undergoes activation via a heat treatment and/or a fluorination treatment, the activation possibly taking place during the fluorination.

5. Process according to one of Claims 1 to 4, **characterized in that** the catalyst is a chromium III oxide that has optionally undergone activation via a fluorination treatment.

6. Process according to one of Claims 1 to 5, **characterized in that** the active phase is provided in a finely divided form or is formed or deposited on a support.

7. Process according to Claim 6, **characterized in that** the chromium is deposited on a support in a proportion of 0.5% to 5% of the weight of the catalyst.

8. Process according to one of Claims 1 to 7, **characterized in that** the ratio between the number of moles of hydrofluoric acid and the number of moles of dichloroacetyl chloride ranges between 1 and 30.

9. Process according to one of Claims 1 to 8, **characterized in that** the reaction temperature is between 250°C and 400°C.

10. Process according to one of Claims 1 to 9, **characterized in that** the process is performed in batch or continuous mode.

11. Process according to one of Claims 1 to 10, **characterized in that** the reaction is performed in continuous mode, in a tubular reactor comprising the solid catalyst arranged as a fixed bed.

12. Process according to one of Claims 1 to 11, **characterized in that** the dichloroacetyl chloride and the hydrofluoric acid are mixed together in a mixing zone, and the mixture obtained is then conveyed onto the catalytic bed.

13. Process according to one of Claims 1 to 12, **characterized in that** the contact time, defined as the ratio between the apparent volume of catalyst and the flow rate of the gas stream, ranges between 0.2 and 100 seconds.

14. Process according to one of Claims 1 to 13, **characterized in that** the weight of substrate used per weight of catalyst and per hour ranges between 0.01 h⁻¹ and 2 h⁻¹.

15. Process according to one of Claims 1 to 14, **characterized in that**, at the end of the reaction, a gaseous phase comprising the difluoroacetyl fluoride, the excess hydrofluoric acid and the hydrochloric acid formed by the reaction is recovered.

16. Process according to one of Claims 1 to 15, **characterized in that** a step of hydrolysis of the difluoroacetyl fluoride to difluoroacetic acid is then performed by placing the gas stream in contact with water.

17. Process according to one of Claims 1 to 16, **characterized in that** the difluoroacetic acid obtained is reacted with a base.

18. Process for preparing difluoroacetyl fluoride, **characterized in that** it is obtained by reacting dichloroacetyl chloride and hydrofluoric acid, in the gas phase, in the presence of a chromium-based catalyst, according to one of Claims 1 to 15.
